# EUROPEAN PATENT APPLICATION

(11) **EP 4 111 952 A1**
(43) Date of publication of application: **04.01.2023**
(21) Application number: 22737703.3
(22) Date of filing: 21.01.2022
(51) Int. Cl.: A61B 5/00, A61B 5/145, G16H 50/20, G16H 40/67, A61B 1/24

(54) **TOOTH ANALYSIS SERVER, TOOTH ANALYSIS TERMINAL, AND TOOTH ANALYSIS PROGRAM**

(30) Priority: 17.02.2021 KR 20210021397; 09.11.2021 KR 20210152724
(71) Applicant: Iclo. Ltd, Jeju-si Jeju-do 63169 (KR)
(72) Inventor: KIM, Jun Bae, Gimpo-si, Gyeonggi-do 10079 (KR)
(74) Representative: Dr. Gassner & Partner mbB
(86) International application number: PCT/KR2022/001089
(87) International publication number: WO 2022/177181

(57) **Abstract**

Provided is a tooth analysis server including: a server communication unit linked with a tooth photographing device for photographing teeth to communicate with a user terminal; a database management unit for managing an image of the photographed teeth as data; an analysis unit for analyzing a state of the teeth of the user according to the request; and a result providing unit for providing an analysis result for the state of the teeth of the user to the user terminal, wherein the analysis unit receives a first image obtained by photographing the teeth of the user at a first speed from the tooth photographing device to perform a first analysis, and receives a second image obtained by photographing the doubtful area at a second speed slower than the first speed from the tooth photographing device to perform a second analysis.

## Description

### BACKGROUND OF THE INVENTION

### 1. Field of the invention

The present invention relates to a tooth analysis server, a tooth analysis terminal, and a tooth analysis program, and more specifically, to a tooth analysis server, a tooth analysis terminal, and a tooth analysis program to self-examine teeth and treat the teeth at a low cost.

### 2. Description of the Prior Art

It may be difficult for an average person to analyze a dental condition at home without visiting a dentist.

Accordingly, most ordinary people visit the dentist when they feel uncomfortable with the teeth.

From another point of view, this may signify that the dental disease has already progressed to the degree that the ordinary person feels uncomfortable with the teeth.

As in the above, when the person with already advanced dental disease visits the dentist, the range of treatment may be widened, the period of treatment may be prolonged, and the cost of treatment may be increased.

Therefore, there is a need for a dental analysis system capable of easily diagnosing dental diseases in the early stage at a home level before visiting the dentist.

### SUMMARY OF THE INVENTION

The technical problem to be solved by the present invention is to provide a tooth analysis server, a tooth analysis terminal, and a tooth analysis program to self-examine teeth of a user at a home level.

Another technical problem to be solved by the present invention is to provide a tooth analysis server, a tooth analysis terminal, and a tooth analysis program to enable a user to receive a dental treatment at a low cost in a hospital when the user has a tooth having dental disease as a result of the self-examination.

The technical problems to be solved by the present invention are not limited to the above description.

In order to solve the above-mentioned technical problems, the present invention provides a tooth analysis server.

According to one embodiment, the tooth analysis server includes a server communication unit linked with a tooth photographing device for photographing teeth to communicate with a user terminal, a database management unit for managing an image of the photographed teeth as data, an analysis unit configured, when an analysis for the teeth of the user is requested from the user terminal through the server communication unit, to analyze a state of the teeth of the user according to the request based on an artificial intelligence learning result learned from the data managed by the database management unit, and a result providing unit for providing an analysis result for the state of the teeth of the user to the user terminal, wherein the analysis unit, when the analysis for the teeth of the user is requested, may receive a first image obtained by photographing the teeth of the user at a first speed from the tooth photographing device to perform a first analysis, may, when there is a doubtful area suspected of a dental disease in a specific tooth among the user's teeth as a result of the first analysis, receive a second image obtained by photographing the doubtful area at a second speed slower than the first speed from the tooth photographing device to perform a second analysis.

According to one embodiment, the tooth analysis server further includes a photographing assistant unit. The photographing assistant unit may provide the user terminal with an assistant photographing area for assisting the photographing such that the analysis unit analyzes the doubtful area being photographed using the tooth photographing device, when the second image is photographed using the tooth photographing device.

According to one embodiment, the tooth analysis server further includes an advertisement transmitting unit, and the advertisement transmitting unit may transmit an advertisement to the user terminal during analyzing a state of the user's teeth by the analysis unit.

According to one embodiment, when the analysis unit analyzes that a specific tooth has a dental disease as a result of the second analysis, the result providing unit may provide an expected treatment amount for treating the dental disease to the user terminal. The tooth analysis server further includes a hospital recommendation unit, and the hospital recommendation unit may provide hospital information for treating the disease with the expected treatment amount to the user terminal.

In order to solve the above-mentioned technical problems, the present invention provides a dental analysis device.

According to one embodiment, the tooth analysis terminal includes a terminal communication unit for communicating with at least one of a tooth photographing device for photographing teeth, and a tooth analysis server for analyzing a state of the user's teeth based on an artificial intelligence learning result, and a display unit for outputting an analysis result obtained by analyzing the image of the user's teeth photographed through the tooth photographing device, by the artificial intelligence learning result, when an analysis of the user's teeth is requested from the user, wherein the display unit may output a first analysis result obtained by analyzing a first image of the user's teeth photographed at a first speed is analyzed by the artificial intelligence learning result, and may further output a second analysis result obtained by analyzing a second image, in which the doubtful area is photographed at a second speed slower than the first speed, by the artificial intelligence learning result, when there is a doubtful area suspected of a dental disease in a specific tooth among the user's teeth as a result of the first analysis.

In order to solve the above-mentioned technical problems, the present invention provides a dental analysis program.

According to one embodiment, the tooth analysis program may be stored in a medium to execute the steps of: receiving a request for analysis of teeth of a user from the user; receiving a first image of the user's teeth photographed at a first speed; providing the user with a first analysis result in which the first image is analyzed by an artificial intelligence learning result; receiving a second image when there is a doubtful area suspected of a dental disease in a specific tooth among the user's teeth as a result of the first analysis, in which the doubtful area is photographed at a second speed slower than the first speed; and providing the user with a second analysis result in which the second image is analyzed by the artificial intelligence learning result.

The embodiment of the present invention provides the a tooth analysis server including: a server communication unit linked with a tooth photographing device for photographing teeth to communicate with a user terminal, a database management unit for managing an image of the photographed teeth as data, an analysis unit configured, when an analysis for the teeth of the user is requested from the user terminal through the server communication unit, to analyze a state of the teeth of the user according to the request based on an artificial intelligence learning result learned from the data managed by the database management unit, and a result providing unit for providing an analysis result for the state of the teeth of the user to the user terminal, wherein the analysis unit when the analysis for the teeth of the user is requested may receive a first image obtained by photographing the teeth of the user at a first speed from the tooth photographing device to perform a first analysis, and may, when there is a doubtful area suspected of a dental disease in a specific tooth among the user's teeth as a result of the first analysis, receive a second image obtained by photographing the doubtful area at a second speed slower than the first speed from the tooth photographing device to perform a second analysis.

In addition, according to the embodiment of the present invention, when the analysis unit analyzes that a specific tooth has a dental disease as a result of the second analysis, the result providing unit may provide an expected treatment amount for treating the dental disease to the user terminal. The tooth analysis server further includes a hospital recommendation unit, and the hospital recommendation unit may provide hospital information for treating the disease with the expected treatment amount to the user terminal.

Thus, according to one embodiment of the present invention, the user can self-examine the user's teeth at a home level, and receive a dental treatment at a low cost in a hospital when the user has a tooth having dental disease as a result of the self-examination.

### BRIEF DESCRIPTION OF THE DRAWINGS

The above and other objects, features, and advantages of the present invention will be more apparent from the following detailed description taken in conjunction with the accompanying drawings, in which:
FIGS. 1 and 2 are diagrams for explaining a tooth analysis system according to the embodiment of the present invention.
FIG. 3 is a diagram for explaining a tooth analysis terminal according to the embodiment of the present invention.
FIG. 4 is a diagram for explaining a tooth analysis server according to the embodiment of the present invention.
FIGS. 5 to 18 are diagrams for explaining a tooth analysis method according to the embodiment of the present invention.

### DETAILED DESCRIPTION OF THE EXEMPLARY EMBODIMENTS

Hereinafter, exemplary embodiments of the present disclosure will be described in detail with reference to the accompanying drawings. However, the technical idea of the present disclosure is not limited to the embodiments, but may be realized in different forms. The embodiments introduced herein are provided to sufficiently deliver the idea of the present disclosure to those skilled in the art so that the disclosed contents may become thorough and complete.

When it is mentioned in the present disclosure that one element is on another element, it means that a first element may be directly formed on a second element, or a third element may be interposed between the first element and the second element. Further, in the drawings, thicknesses of membranes and areas are exaggerated for efficient description of the technical contents.

In addition, in the various embodiments of the present disclosure, the terms such as first, second, and third are used to describe various elements, but the elements are not limited to the terms. The terms are used only to distinguish one element from another element. Therefore, an element mentioned as a first element in one embodiment may be mentioned as a second element in another embodiment. The embodiments described and illustrated herein include their complementary embodiments. Further, the term "and/or" used herein is used to include at least one of the elements enumerated before and after the term.

As used herein, the terms of a singular form may include plural forms unless the context clearly indicates otherwise. Further, the terms such as "including" and "having" are used to designate the presence of features, numbers, steps, elements, or combinations thereof described in the present disclosure, and shall not be construed to preclude any possibility of presence or addition of one or more other features, numbers, steps, elements, or combinations thereof. In addition, the term "connection" used herein is used to include both indirectly and directly connecting a plurality of elements.

Further, in the following description of the present disclosure, detailed descriptions of known functions and configurations incorporated herein will be omitted when they may make the subject matter of the present disclosure unnecessarily unclear.

Hereinafter, a dental analysis system according to the embodiment of the present invention will be described with reference to the drawings.

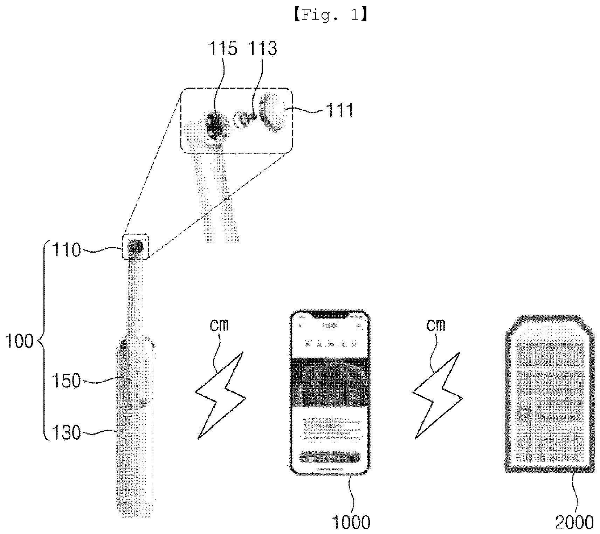

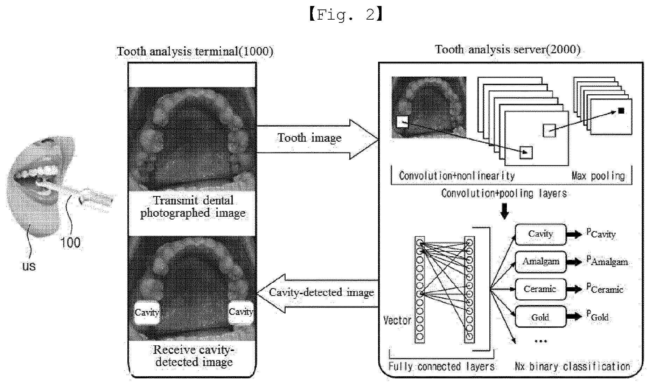

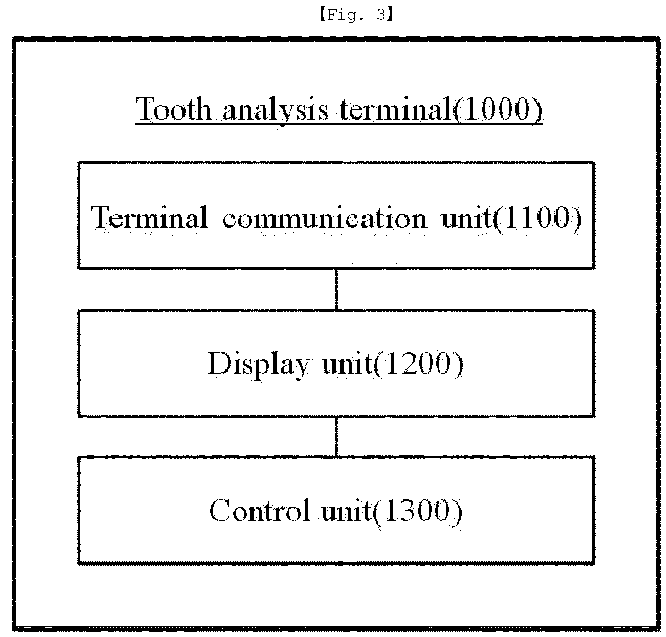

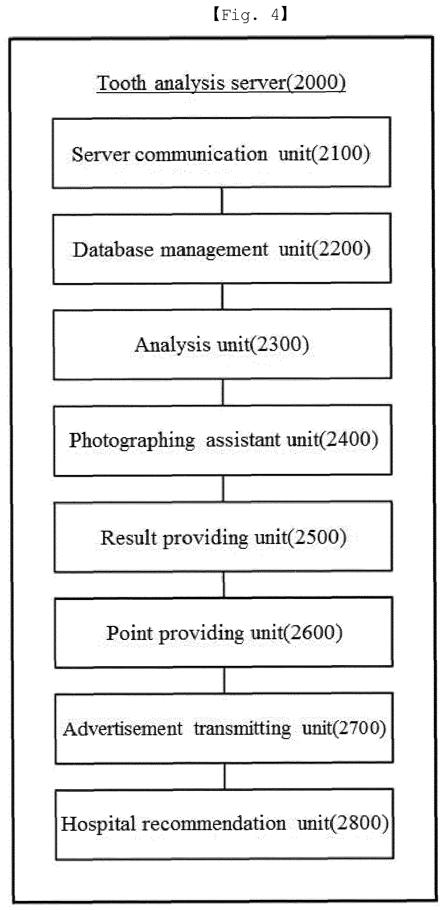

FIGS. 1 and 2 are diagrams for explaining a tooth analysis system according to the embodiment of the present invention. FIG. 3 is a diagram for explaining a tooth analysis terminal according to the embodiment of the present invention. FIG. 4 is a diagram for explaining a tooth analysis server according to the embodiment of the present invention.

According to the dental analysis system of the embodiment of the present invention, the user can self-examine the user's teeth at a home level, and receive a dental treatment at a low cost in a hospital when the user has a tooth having dental disease as a result of the self-examination.

To this end, referring to FIG. 1, the tooth analysis system may be understood as a concept including at least one of a tooth photographing device 100, a tooth analysis terminal 1000, and a tooth analysis server 2000.

According to one embodiment, the tooth photographing device 100 and the tooth analysis terminal 1000 may be linked to each other. In addition, the tooth analysis terminal 1000 and the tooth analysis server 2000 may be linked to each other. The term "link" may be understood as a concept including communication (cm, see FIG. 1) between the above-described components.

Accordingly, referring to FIG. 2, when a user us photographs the user's teeth using the tooth photographing device 100, the tooth analysis terminal 1000 may receive an image of the user's teeth from the tooth photographing device 100 and provide the received image to the tooth analysis server 2000, and may receive an analysis result obtained by analyzing a state of the user's teeth from the tooth analysis server 2000 and output the received analysis result on a screen.

Thus, according to the embodiment of the present invention, dental diseases can be easily diagnosed in the early stage at a home level before visiting the dentist.

Accordingly, the dental disease can be early treated, and the cost of treatment can be reduced.

Hereinafter, each configuration will be described.

### Tooth photographing device 100

Referring to FIG. 1, the tooth photographing device 100 may include at least one of a photographing unit 110, a body unit 130, an operation unit 150, and a device communication unit (not shown).

Hereinafter, each configuration will be described.

### Photographing unit 110

The photographing unit 110 may photograph the user's teeth as an image. The term "image" may be understood as a concept including both a still image and a moving image.

To this end, as shown in FIG. 1, the photographing unit 110 may be configured to include at least one of a lens 111 that collects or diverges light to form an optical image, an image sensor 113 converting the light passing through the lens 111 into an electrical digital signal to output the electrical digital signal through a display unit 1200 (see FIG. 3) of the tooth analysis terminal 1000 described later, and a light emitting unit 115 for providing light to a photographing area.

### Body unit 130

The body unit 130 may be provided to be held in the user's hand when the user photographs the user's teeth as an image through the photographing unit 110.

To this end, the body unit 130 may have a width and a height that can be held in the user's hand.

### Operation unit 150

The operation unit 150 may be provided to enable the user to drive the photographing unit 110. The term "drive" may be understood as a concept including movements for photographing the user's teeth as an image through the photographing unit 110 by the user, for example, movements according to power, such as turning on and off power of the photographing unit 110, starting the photographing of the photographing unit 110, or irradiating light from the light emitting unit 115.

To this end, the operation unit 150, for example, may be provided in the form of an operation button or a touch switch.

### Device communication unit (not shown)

The device communication unit (not shown) may provide the image of the user's teeth photographed through the photographing unit 110 to the tooth analysis terminal 1000 described later.

To this end, the device communication unit (not shown) may communicate with a terminal communication unit 1100 (see FIG. 3) of the tooth analysis terminal 1000 described later.

### Tooth analysis terminal 1000

Referring to FIG. 3, the tooth analysis terminal 1000 may include at least one of a terminal communication unit 1100, a display unit 1200, and a control unit 1300.

The tooth analysis terminal 1000 may be understood as a concept including a user terminal, such as a smart phone and a tablet personal computer (hereinafter, the tooth analysis terminal 1000 and the user terminal are defined as the same concept).

Hereinafter, each configuration will be described.

### Terminal communication unit 1100

The terminal communication unit 1100 may obtain the image of the user's teeth photographed through the tooth photographing device 100, and provide the obtained image to the tooth analysis server 2000 described later.

To this end, the terminal communication unit 1100 may communicate with at least one of the device communication unit (not shown) of the tooth photographing device 100, and a server communication unit 2100 of the tooth analysis server 2000 described later.

### Display unit 1200

When an analysis of the user's teeth is requested from the user, the display unit 1200 may output at least one of the image of the user's teeth photographed through the tooth photographing device 100, and the analysis result obtained by analyzing the image of the tooth by the tooth analysis server 2000 described later.

To this end, the display unit 1200 may be provided in the form of a screen.

According to one embodiment, the display unit 1200 may be provided in the form of a touch screen through which the user may apply a touch input.

Accordingly, the display unit 1200 may enable manipulation by touch necessary for analyzing the user's teeth as described above.

### Control unit 1300

The control unit 1300 may perform overall control for the tooth analysis terminal 1000.

More specifically, the control unit 1300 may control the communication of the terminal communication unit 1100, and may control the screen output of the display unit 1200.

In addition, according to one embodiment, the control unit 1300 may control each unit such that the user's touch input through the display unit 1200 is executed. For example, the control unit 1300 may control to output the first analysis result of the tooth analysis server 2000 described later through the display unit 1200, in which it may be determined whether the second analysis proceeds, based on the first analysis result. This is assuming that the user has determined whether to perform the second analysis based on the first analysis result outputted on the display unit 1200.

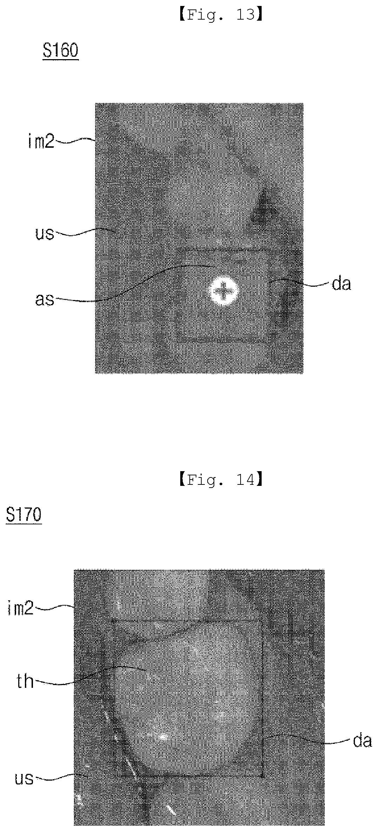

Alternatively, for another example, the control unit 1300 may control such that the assistant photographing area as (see FIG. 13) for the second analysis of the tooth analysis server 2000 described later is outputted through the display unit 1200. This will be described later in more detail.

### Tooth analysis server 2000

Referring to FIG. 4, the tooth analysis server 2000 may include at least one of a server communication unit 2100, a database management unit 2200, an analysis unit 2300, a photographing assistant unit 2400, a result providing unit 2500, a point providing unit 2600, an advertisement transmitting unit 2700, and a hospital recommendation unit 2800.

Hereinafter, each configuration will be described.

Server communication unit 2100 the terminal communication unit 2100 may communicate with the terminal communication unit 1100 to obtain the image of the user's teeth photographed through the tooth photographing device 100.

In addition, as described above, the terminal communication unit 1100 may communicate with at least one of the device communication unit (not shown) of the tooth photographing device 100, and the server communication unit 2100, so that the terminal communication unit 2100 may obtain, that is, receive the image of the user's teeth through the terminal communication unit 1100.

### Database management unit 2200

The database management unit 2200 may manage the image of the photographed teeth as data.

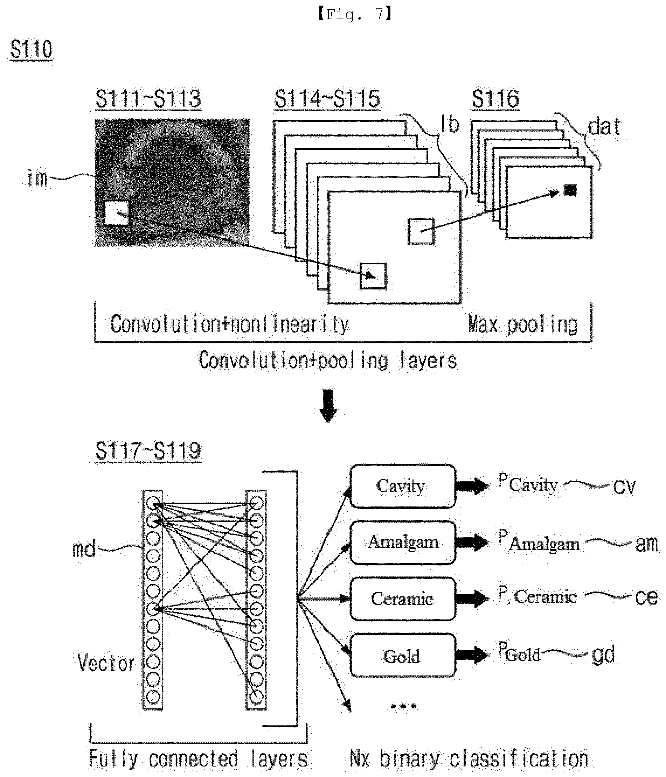

More specifically, referring to FIG. 7, the database management unit 2200 may accumulate images im of the photographed teeth and manage the accumulated image as data dat.

For example, as shown in FIG. 7, the database management unit 2200 may accumulate images of teeth according to states of the teeth formed with cavities cv, amalgam am, ceramics ce, gold gd and the like and manage the accumulated images as data dat.

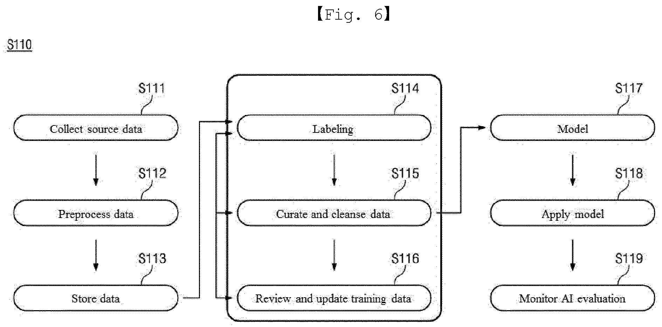

To this end, referring to FIG. 6, the database management unit 2200 may collect source data, pre-process the source data, and store the preprocessed data.

The source data may refer to the images im (see FIG. 7) of the teeth photographed through the tooth photographing device 100. Alternatively, according to one embodiment, the source data may be data provided from the outside for learning in the analysis unit 2300 described later. The data provided from the outside may be understood as a concept including a tooth image other than the image of the tooth photographed through the tooth photographing device 100. At this point, the image of the data provided from the outside may also include both a still image and a moving image.

In addition, according to one embodiment, the database management unit 2200 may label the stored data (lb, see FIG. 7), data-curate and cleanse the labeled data, and review and update the data-curated and cleansed data as learning data dat (see FIG. 7).

As shown in FIG. 7, the labeling lb (see FIG. 7) may refer to labeling the stored data according to a tooth state, for example, according to a state of the tooth formed with cavities cv, amalgam am, ceramics ce, gold gd, and the like.

In addition, the data curation may be understood as a concept including classifying the labeled data lb (see FIG. 7) according to the state of the teeth.

In addition, according to one embodiment, the database management unit 2200 may generate a model md (see FIG. 7) from the reviewed and updated data as the learning data to learn in the analysis unit 2300 described later. In addition, the database management unit 2200 may apply the model md (see FIG. 7) and use the applied model to monitor an artificial intelligence (AI) learning evaluation of the analysis unit 2300 described later.

Accordingly, referring to FIG. 7, the analysis unit 2300 described later may analyze (ans) the state of the user's teeth according to the learning result learned by the artificial intelligence based on the model md generated by the database management unit 2200.

### Analysis unit 2300

When an analysis for the teeth of the user is requested from the tooth analysis terminal 1000 through the server communication unit 2100, the analysis unit 2300 may analyze the state of the user's teeth.

To this end, the analysis unit 2300 may learn the model md (see FIG. 7) generated by the database management unit 2200 through the artificial intelligence as described above so as to analyze the state of the user's teeth.

Accordingly, the analysis unit 2300 can analyze the state of the user's teeth based on the artificial intelligence learning result.

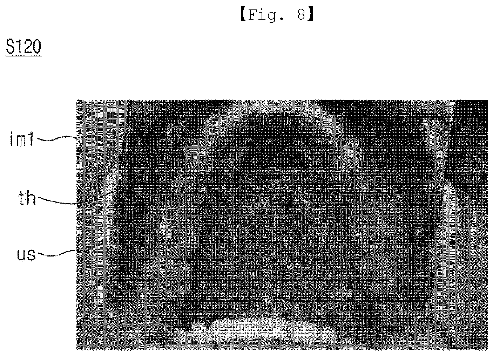

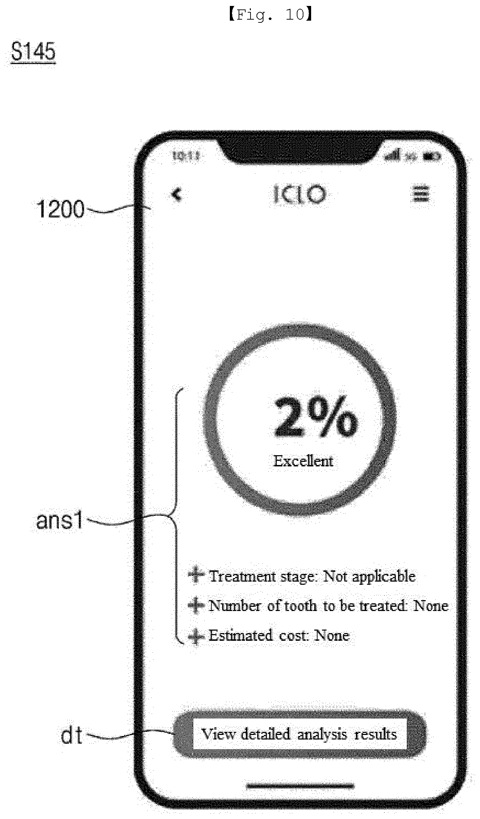

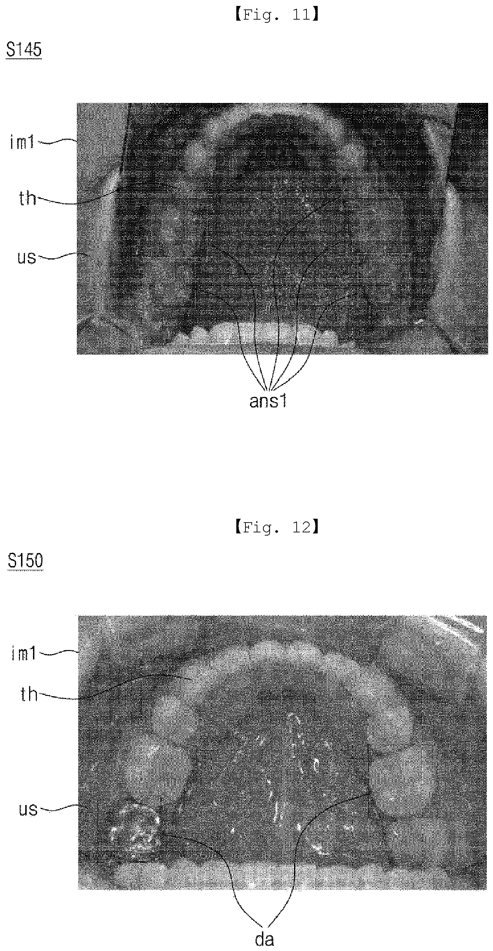

In addition, when the analysis for the user's teeth is requested from the tooth analysis terminal 1000, the analysis unit 2300 may receive a first image im1 obtained by photographing the teeth th of the user us at a first speed from the tooth photographing device 100 as shown in FIG. 8, so that the first analysis (ans1) may be performed as shown in FIGS. 10 and 11. This is assuming that the user us has photographed the teeth th of the user us through the tooth photographing device 100.

According to one embodiment, the analysis unit 2300 may analyze that the teeth of the user us do not have a doubtful area suspected of having a dental disease, as a result of the first analysis ans1.

Accordingly, the result providing unit 2500 described later may provide, as a text, the result of the first analysis ans1 analyzed that there is no doubtful area suspected of the dental disease through the display unit 1200 of the tooth analysis terminal 100 as shown in FIG. 10, and may also provide an image as shown in FIG. 11. This will be described later in more detail.

According to another embodiment, when the analysis unit 2300 analyzes that a doubtful area da, which is suspected of a dental disease, is present in a specific tooth among the teeth th of the user us as shown in FIG. 12, as a result of the first analysis, the analysis unit 2300 may receive a second image im2 (see FIG. 14), in which the doubtful area da is photographed at a second speed slower than the first speed from the tooth photographing device 100, thereby perform a second analysis. This is assuming that the user us has photographed the teeth th of the user us through the tooth photographing device 100. In addition, the second speed slower than the first speed may be understood as a concept including a stationary state (in other words, a still image is photographed).

During the second analysis as described above, the photographing at the second speed slower than the first speed upon the first analysis is for analyzing the doubtful area da more precisely because the doubtful area da as described above is present. In other words, the above-described first analysis may serve as a concept of examining the entire teeth of the user us by scanning the entire teeth of the user us at a high speed, and the second analysis is configured to limit the above-described doubtful area da to precisely analyze the doubtful area da.

In addition, the doubtful area da is required to be photographed at a level that can be analyzed, so that in the analysis unit 2300 precisely analyzes the doubtful area da as described above, because it is assuming, as described above, that the user us, that is, a person photographs teeth through the tooth photographing device 100. In addition, the user us may be a concept including an ordinary person, that is, a non-expert other than an expert such as a dentist. Accordingly, it is taken into account that the non-expert may be unskilled at photographing teeth for analysis.

Thus, the present invention provides a photographing assistant unit 2400 described later.

### Photographing assistant unit 2400

Referring to FIG. 13, when the user us photographs the second image im2 using the tooth photographing device 100, the photographing assistant unit 2400 may provide an assistant photographing area as for assisting the photographing, through the display unit 1200 of the tooth analysis terminal 1000.

For example, as shown in FIG. 13, the photographing assistant unit 2400 may provide the assistant photographing area as an area filled with red color so that the doubtful area da is photographed.

Accordingly, the user us may photograph the teeth while moving the tooth photographing device 100 in the oral cavity, so that the user's teeth th (see FIG. 14) are positioned in the assistant photographing area as filled with the red color.

Accordingly, as shown in FIG. 14, the analysis unit 2300 may obtain the second image im2 for analyzing the doubtful area da. This is assuming that the doubtful area da in the second image im2 is photographed at a level that can be analyzed as described above.

At this point, the control unit 1300 may control to provide a user interface through the display unit 1200.

Accordingly, the user us may view the doubtful area da through the user interface while manipulating the doubtful area through an enlarged screen and/or reduced screen by a hand, and may re-photograph the image such that the user's teeth th are positioned in the assistant photographing area as when the user's tooth th shown in FIG. 14 is not positioned in the assistant photographing area as shown in FIG. 13.

Accordingly, the analysis unit 2300 may precisely analyze the doubtful area da based on the second image im2 photographed at a level capable of analyzing the doubtful area da.

### Result providing unit 2500

The result providing unit 2500 may provide the analysis result on the state of the user's teeth to the tooth analysis terminal 1000.

According to one embodiment, as shown in FIG. 10, when the analysis unit 2300 performs the first analysis ans1 that there is no doubtful area suspected of the dental disease as described above, the result providing unit 2500 may provide the result of the first analysis ans1 as information, through the display unit 1200 of the tooth analysis terminal 100, for example, the percentage (%) of cavity progression as 2%, the treatment stage as Not applicable, the number of tooth to be treated as None, and the estimated cost (that is, estimated amount of treatment) as None.

The analysis unit 2300, as shown in FIG. 10, may provide a touch input button for viewing detailed analysis results dt so as to view the results of the first analysis ans1 in more detail, through the display unit 1200.

Accordingly, when the user touches the touch input button for viewing detailed analysis results dt, the analysis unit 2300 may display the analysis result on the first image im1, for example, by providing a green line ans1 as an image through the display unit 1200 (see FIG. 11).

The control unit 1300 may control the display unit 1200 to provide a user interface.

Accordingly, the user us may view the green line ans1 (see FIG. 11) through the user interface while manipulating the green line through an enlarged screen and/or reduced screen by a hand.

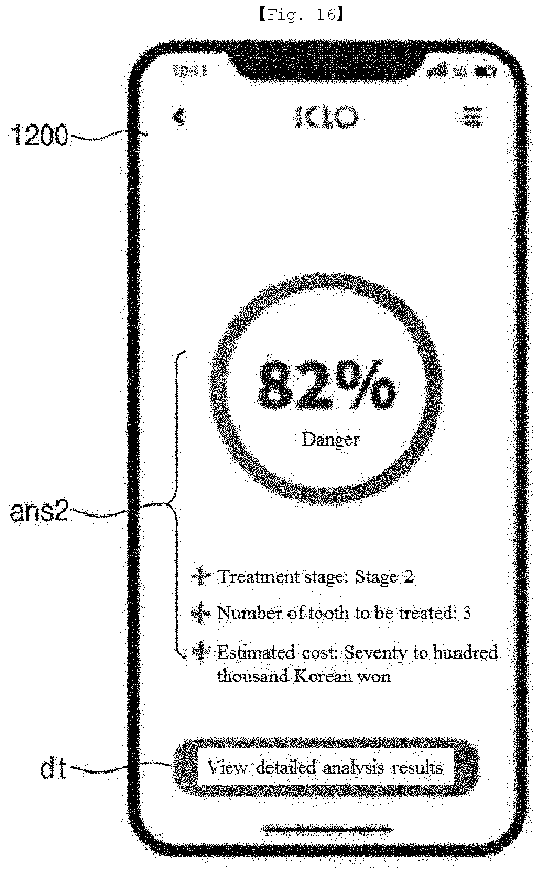

Alternatively, in another embodiment, as previously described with reference to FIG. 12, when the analysis unit 2300 performs the first analysis that there is a doubtful area da suspected of the dental disease and accordingly performs the second analysis, the result providing unit 2500 may also provide the result on the second analysis ans2 as information through the display unit 1200 of the tooth analysis terminal 100, for example, the percentage (%) of cavity progression as 82%, the treatment stage as Stage 2, the number of tooth to be treated as 3, and the estimated cost (that is, estimated amount of treatment) as 70,000 Korean won to 100,000 Korean won, as shown in FIG. 16.

As described with reference to FIG. 10, the analysis unit 2300 may provide a touch input button for viewing detailed analysis results dt so as to view the results of the second analysis ans2 in more detail through the display unit 1200 as shown in FIG. 16.

Accordingly, when the user touches the touch input button for viewing detailed analysis results dt, the analysis unit 2300 may display and provide the analysis result on the second image im2 through the display unit 1200 as shown in FIG. 14. The control unit 1300 may control the display unit 1200 to provide a user interface. This is the same as the description of the result providing unit 2500 providing the first analysis result as described above, and may refer to the above-described embodiment.

### Point providing unit 2600

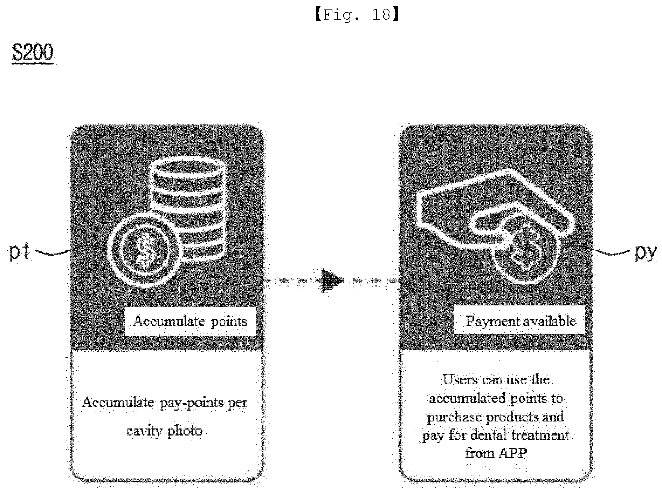

Referring to FIG. 18, when the user provides the tooth analysis server 2000 with at least one of the first and second analysis results provided from the analysis unit 2300 the point providing unit 2600 may provide points pt for payment py. The points pt for payment py may be understood as a concept of points pt for purchasing a device according to an embodiment such as the tooth photographing device 100 (see FIG. 1). In this case, the points pt may be understood as a concept so as to be used like cash upon purchasing the device as described above and deducted at the time of payment py. Herein, the points pt for payment py may also be understood as a concept of points pt for payment py for a hospital bill in a hospital recommended by the hospital recommendation unit 2800 described later. In this case, the points pt may be used like cash upon payment py of the hospital bill and deducted at the time of payment py.

According to the embodiment of the present invention, this is considered for artificial intelligence learning in the analysis unit 2300.

As described above, the analysis unit 2300 may analyze (ans) the state of the user's teeth according to the learning result learned by the artificial intelligence based on the model md generated by the database management unit 2200.

For the above process, when the user provides the tooth analysis server 2000 with at least one of the first and second analysis results provided from the analysis unit 2300 as described above, the database management unit 2200 may accumulate the dental images according to the state of the teeth for the artificial intelligence learning and manage the accumulated dental images as data.

Accordingly, the analysis unit 2300 may perform more artificial intelligence learning based on the above-described accumulated data, and accordingly, analyze the teeth more accurately.

### Advertisement transmitting unit 2700

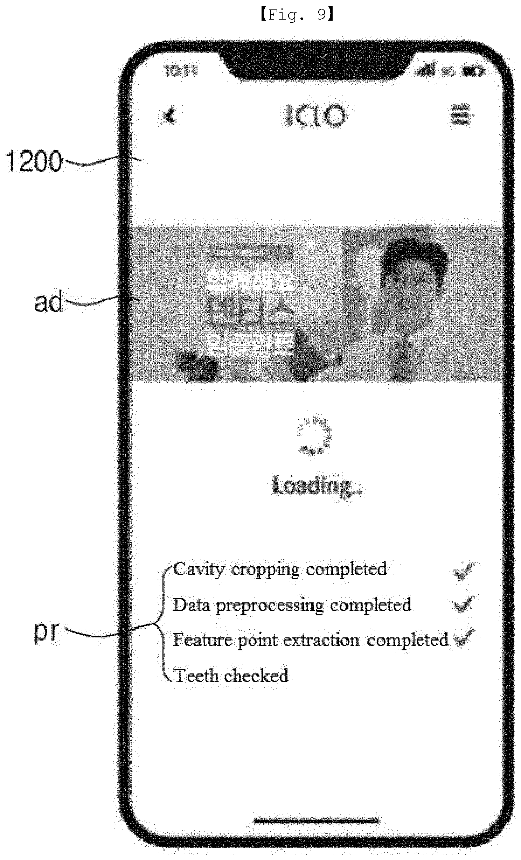

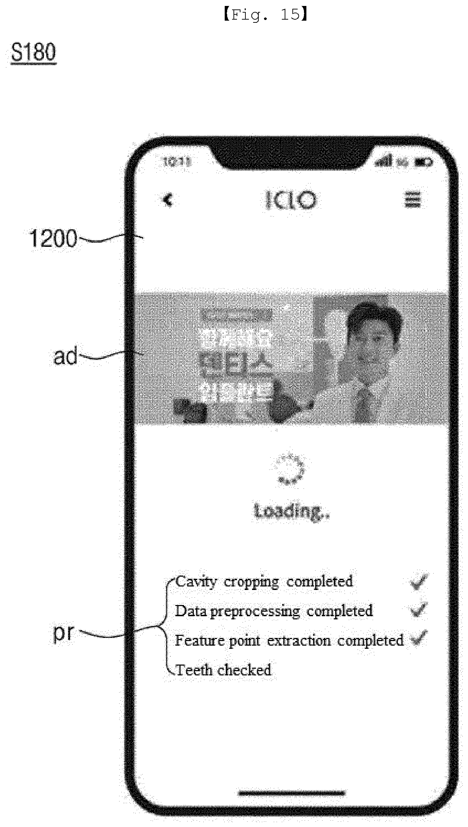

Referring to FIGS. 9 and 15, the advertisement transmitting unit 2700 may transmit an advertisement ad to the tooth analysis terminal 1000 during analyzing a state of the user's teeth by the analysis unit 2300.

The transmitting of the advertisement ad may include at least one of a concept of transmitting advertisements from the tooth analysis server 2000 according to the embodiment of the present invention, and a concept of transmitting advertisements provided from the outside. In addition, the term "outside" may be understood as a concept including an external advertisement providing unit provided separately from the tooth analysis server 2000.

To this end, the above-described control unit 1300 of the tooth analysis terminal 1000 may control the advertisements ad to be outputted through the display unit 1200.

In addition, according to one embodiment, the analysis unit 2300 may provide an analysis process pr through the display unit 1200 while the advertisement ad is being outputted. The analysis process pr may refer to the degree of performing the analysis on the user's tooth state by the analysis unit 2300, for example, the degree of analysis performance related to whether the user's teeth are being analyzed and/or whether the analysis is completed as shown in FIGS. 9 and 15.

### Hospital recommendation unit 2800

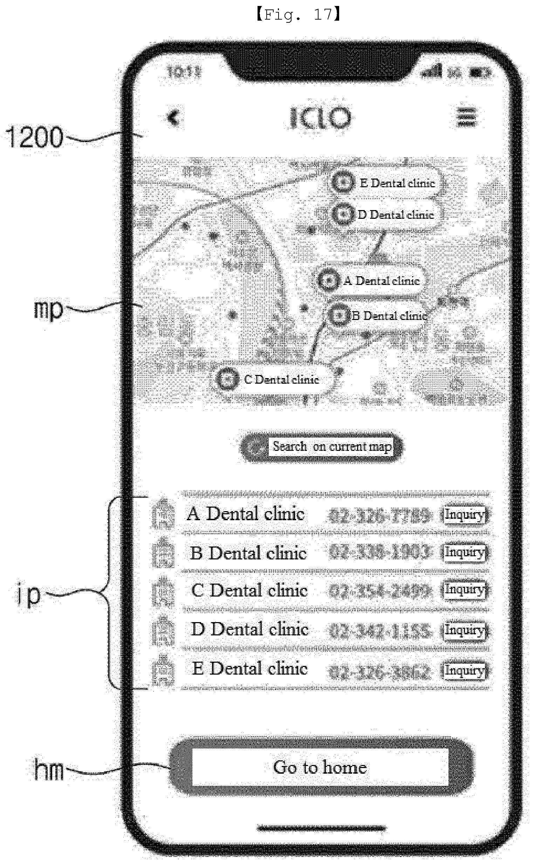

Referring to FIG. 17, when the analysis unit 2300 analyzes that a specific tooth has a dental disease as a result of the second analysis, the hospital recommendation unit 2800 may provide hospital information for treating the disease within the expected treatment amount to the tooth analysis terminal 1000.

To this end, the hospital recommendation unit 2800 may inquire about the presence of a hospital capable of treating the disease within the expected treatment amount analyzed by the analysis unit 2300, based on nearby hospitals around the user's location. The user's location may be understood as a concept including at least one of a user's current location and a location set by the user.

When the hospital capable of treating the disease within the expected treatment amount exists as a result of inquiring about whether treatment is available at nearby hospitals as described above, the hospital recommendation unit 2800 may receive treatment-available reply information from the treatment-available hospitals.

In this case, as shown in FIG. 17, the hospital recommendation unit 2800 may provide the user with information ip on the treatment-available hospitals that provides the treatment-available reply information through the display unit 1200. The information ip on the treatment-available hospitals may be provided with at least one piece of information. When a plurality of pieces of information are provided, a hospital list may be provided as shown in FIG. 17.

Referring to FIG. 17, the hospital recommendation unit 2800 may provide a map mp for guiding a location of any one hospital selected by the user from the hospital list outputted through the display unit 1200. The guiding a hospital location may be understood as a concept including guiding a route, distance, time, and the like from the user's location to the selected hospital.

Thus, according to one embodiment of the present invention, the user can self-examine the user's teeth at a home level, and receive a dental treatment at a low cost in a hospital when the user has a tooth having dental disease as a result of the self-examination.

The dental analysis system according to the embodiment of the present invention have been described.

Hereinafter, a teeth analysis method according to the embodiment of the present invention will be described. For the tooth analysis method described below, the description overlapping with the above-described tooth analysis system may be omitted. However, the present invention does not exclude the omission of overlapping description below. In addition, the tooth analysis method described below may be implemented by the above-described tooth analysis system, that is, at least any one of the tooth photographing device 100, the tooth analysis terminal 1000, and the tooth analysis server 2000.

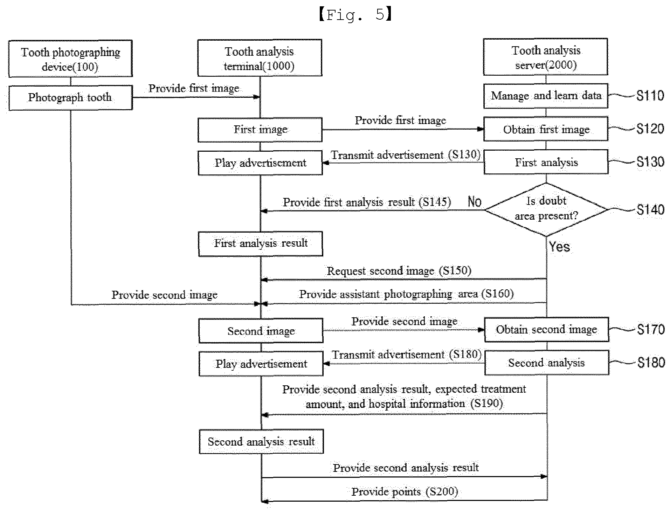

FIGS. 5 to 18 are diagrams for explaining the teeth analysis method according to the embodiment of the present invention.

Referring to FIG. 5, the teeth analysis method includes at least one of: a data management and learning step S110, a first image acquisition step S120, a first analysis step S130, a doubtful area existence determining step S140, a first analysis result providing step S145, a second image request step S150, an assistant photographing area providing step S160, a second image acquisition step S170, a second analysis step S180, a second analysis result providing step S190, and a point providing step S200.

Hereinafter, each step will be described.

### Step S110

In step S110, the database management unit 2200 may manage the image of the photographed teeth as data.

To this end, referring to FIG. 6, the database management unit 2200 may perform at least one of: a step Sill of collecting source data, a step S112 of pre-processing the source data, a step S113 of storing the pre-processed data, a step S114 of labeling the stored data (lb, see FIG. 7), a step S115 of datacurating and cleansing the labeled data, a step S116 of reviewing and updating the data-curated and cleansed data as training data dat (see FIG. 7), a step S117 of generating a model md (see FIG. 7) for learning in the analysis unit 2300 based on the reviewed and updated data as the learning data, a step S118 of applying the model md (see FIG. 7), and a step S119 of utilizing the analysis unit 2300 to monitor artificial intelligence learning evaluation.

Refer to the description of the previous embodiment for the description overlapping with the previous embodiment.

Accordingly, referring to FIG. 7, the analysis unit 2300 may learn the model md generated by the database management unit 2200 through the artificial intelligence.

Accordingly, when the analysis for the user's teeth is requested from the tooth analysis terminal 1000 in a step described later, the analysis unit 2300 may analyze a state of the user's teeth based on the artificial intelligence learning result.

### Step S120

Referring to FIG. 8, in step S120, the terminal communication unit 2100 may communicate with the terminal communication unit 1100 to obtain the first image im1 obtained by photographing the teeth th of the user us at a first speed through the tooth photographing device 100. This is assuming that the user has primarily photographed the user's teeth through the tooth photographing device 100, and assuming that the user requests the analysis on the first image im1 through the tooth analysis terminal 1000.

In addition, as described above, the terminal communication unit 1100 may communicate with at least one of the device communication unit (not shown) of the tooth photographing device 100, and the server communication unit 2100, so that the terminal communication unit 2100 may obtain, that is, receive the first image im1 through the terminal communication unit 1100.

Accordingly, the analysis unit 2300 may obtain the first image im1.

### Step S130

In step S130, the analysis unit 2300 may analyze the first image im1.

Meanwhile, as shown in FIG. 9, during analyzing a state of the user's teeth by the analysis unit 2300, the advertisement transmitting unit 2700 may transmit an advertisement ad through the display unit 1200 of the tooth analysis terminal 1000. Alternatively, according to one embodiment, the analysis unit 2300 may provide an analysis process pr through the display unit 1200 while the advertisement ad is being outputted.

Refer to the description of the previous embodiment for the description overlapping with the previous embodiment.

### Step S140

In step S140, as a result of the first analysis, the analysis unit 2300 may determine whether a doubtful area suspected of a dental disease exists in the user's teeth.

Accordingly, when the analysis unit 2300 determines that the doubtful area does not exist in this step, step S145 described later may be performed. However, when it is determined that the doubtful area exists, step S150 described later may be performed.

### Step S145

In step S145, the result providing unit 2500 may provide the result on the first analysis ans1 through the display unit 1200 of the tooth analysis terminal 100.

As described with reference to FIG. 10, the result providing unit 2500 may provide information, for example, the percentage (%) of cavity progression as 2%, the treatment stage as Not applicable, the number of tooth to be treated as None, and the estimated cost (that is, estimated amount of treatment) as None, and a touch input button for viewing detailed analysis results dt, through the display unit 1200. Alternatively, as described above with reference to FIG. 11, the result providing unit 2500 may display the analysis result on the first image im1, for example, by providing a green line ans1 as an image as shown in FIG. 11.

Refer to the description of the previous embodiment for the description overlapping with the previous embodiment.

As described in the previous step, this is assuming that the analysis unit 2300 performs the first analysis ans1 that there is no doubtful area suspected of the dental disease.

### Step S150

Referring to FIG. 12, in step S150, when the analysis unit 2300 analyzes that a doubtful area da, which is suspected of a dental disease, is present in a specific tooth among the teeth th of the user us as a result of the first analysis through the first image im1, the analysis unit 2300 may request the second image im2 (see FIG. 14) to the user. In addition, the second image may refer to an image obtained by photographing the doubtful area da at a second speed slower than the first speed from the tooth photographing device 100 as described above.

During the second analysis as described above, the photographing at the second speed slower than the first speed upon the first analysis is for analyzing the doubtful area da more precisely because the doubtful area da as described above is present. In other words, the above-described first analysis may serve as a concept of examining the entire teeth of the user us by scanning the entire teeth of the user us at a high speed, and the second analysis is configured to limit the above-described doubtful area da to precisely analyze the doubtful area da.

Refer to the description of the previous embodiment for the description overlapping with the previous embodiment.

### Step S160

Referring to FIG. 13, in step S160, the photographing assistant unit 2400 may provide an assistant photographing area as for assisting a photographing when the user us photographs the second image im2 using the tooth photographing device 100, through the display unit 1200 of the tooth analysis terminal 1000.

Accordingly, the user us may photograph the teeth while moving the tooth photographing device 100 in the oral cavity, so that the user's teeth th (see FIG. 14) are positioned in the assistant photographing area as filled with the red color.

In addition, this is taken into consideration that the analysis unit 2300 in a step described later precisely analyzes the doubtful area da as described above.

Refer to the description of the previous embodiment for the description overlapping with the previous embodiment.

### Step S170

Referring to FIG. 14, in step S170, the terminal communication unit 2100 may communicate with the terminal communication unit 1100 to obtain the second image im2 obtained by photographing the teeth th of the user us at a second speed through the tooth photographing device 100. This is assuming that the user has secondarily photographed the user's teeth through the tooth photographing device 100, and assuming that the user requests the analysis on the second image im2 through the tooth analysis terminal 1000.

In addition, as described above, the terminal communication unit 1100 may communicate with at least one of the device communication unit (not shown) of the tooth photographing device 100, and the server communication unit 2100, so that the terminal communication unit 2100 may obtain, that is, receive the second image im2 through the terminal communication unit 1100.

Accordingly, the analysis unit 2300 may obtain the second image im2.

### Step S180

In step S180, the analysis unit 2300 may analyze the second image im2.

Meanwhile, as shown in FIG. 15, the advertisement transmitting unit 2700 may transmit an advertisement ad through the display unit 1200 of the tooth analysis terminal 1000 during analyzing a state of the user's teeth by the analysis unit 2300. Alternatively, according to one embodiment, the analysis unit 2300 may provide an analysis process pr through the display unit 1200 while the advertisement ad is being outputted.

Refer to the description of the previous embodiment for the description overlapping with the previous embodiment.

### Step S190

In step S190, the result providing unit 2500 may provide the result on the second analysis ans2 through the display unit 1200 of the tooth analysis terminal 100.

As described with reference to FIG. 16, the result providing unit 2500 may provide information, for example, the percentage (%) of cavity progression as 82%, the treatment stage as Stage 2, the number of tooth to be treated as 3, and the estimated cost (that is, estimated amount of treatment) as 70,000 Korean won to 100,000 Korean won, and a touch input button for viewing detailed analysis results dt, through the display unit 1200.

Refer to the description of the previous embodiment for the description overlapping with the previous embodiment.

Referring to FIG. 17, in this step, when the analysis unit 2300 analyzes that a specific tooth has a dental disease as a result of the second analysis, the hospital recommendation unit 2800 may provide hospital information for treating the disease within the expected treatment amount to the tooth analysis terminal 1000.

To this end, as described above, the hospital recommendation unit 2800 may inquire about the presence of a hospital capable of treating the disease within the expected treatment amount analyzed by the analysis unit 2300, based on nearby hospitals around the user's location. In addition, when the hospital capable of treating the disease within the expected treatment amount exists as a result of inquiring about whether treatment is available at nearby hospitals as described above, the hospital recommendation unit 2800 may receive treatment-available reply information from the treatment-available hospitals. In this case, as shown in FIG. 17, the hospital recommendation unit 2800 may provide the user with information ip on the treatment-available hospitals that provides the treatment-available reply information through the display unit 1200. The information ip on the treatment-available hospitals may be provided with at least one piece of information. When a plurality of pieces of information are provided, a hospital list may be provided as shown in FIG. 17. Referring to FIG. 17, the hospital recommendation unit 2800 may provide a map mp for guiding a location of any one hospital selected by the user from the hospital list outputted through the display unit 1200.

Refer to the description of the previous embodiment for the description overlapping with the previous embodiment.

Thus, according to one embodiment of the present invention, the user can self-examine the user's teeth at a home level, and receive a dental treatment at a low cost in a hospital when the user has a tooth having dental disease as a result of the self-examination.

Referring to FIG. 17, in this step, the control unit 1300 may control the display unit 1200 to output a touch input button for go-to-home hm.

Accordingly, as previously described in step S120, when the user touches the touch input button for go-to-home hm, a step for obtaining the first image of the user's teeth may be performed from a viewpoint of the tooth analysis terminal 1000 and the tooth analysis server 2000, and a step for photographing the first image of the user's teeth may be performed from a viewpoint of the tooth photographing device 100. Thereafter, at least one of steps S130 to S190 described above may be performed.

### Step S200

In step S200, the point providing unit 2600 may provide the user with points pt for payment py. This is assuming that at least one analysis result among the first and second analysis results provided from the analysis unit 2300 by the user is provided to the tooth analysis server 2000.

Since the points pt for payment py overlaps with the description of the previous embodiment, refer to the description of the previous embodiment.

As previously described, this is considered for the artificial intelligence learning by the analysis unit 2300 in the present invention.

As described above, the analysis unit 2300 may analyze (ans) the state of the user's teeth according to the learning result learned by the artificial intelligence based on the model md generated by the database management unit 2200.

In addition, for the above process, when the at least one analysis result among the first and second analysis results provided from the analysis unit 2300 by the user is provided to the tooth analysis server 2000 as described above, the database management unit 2200 may accumulate the dental images according to the state of the teeth for the artificial intelligence learning and manage the accumulated dental images as data.

Accordingly, the analysis unit 2300 may perform more artificial intelligence learning based on the above-described accumulated data, and accordingly, analyze the teeth more accurately.

The teeth analysis method according to the embodiment of the present invention have been described.

According to the embodiment of the present invention, a dental analysis program (not shown) stored in a medium may be provided in order to execute the tooth analysis method described above.

The tooth analysis program (not shown) may be stored in the medium to implement at least any one of steps S110 to S200 described above. The medium may be, for example, the tooth analysis terminal 1000 described above.

Refer to the descriptions of the previous embodiments for steps S110 to S200.

Although the exemplary embodiments of the present disclosure have been described in detail, the scope of the present disclosure is not limited to a specific embodiment, and should be interpreted by the appended claims. In addition, it should be understood by those of ordinary skill in the art that various changes and modifications can be made without departing from the scope of the present disclosure.

## Claims

1. A tooth analysis server comprising:
a server communication unit linked with a tooth photographing device for photographing teeth to communicate with a user terminal;
a database management unit for managing an image of the photographed teeth as data;
an analysis unit configured, when an analysis for the teeth of the user is requested from the user terminal through the server communication unit, to analyze a state of the teeth of the user according to the request, based on an artificial intelligence learning result learned from the data managed by the database management unit; and
a result providing unit for providing an analysis result for the state of the teeth of the user to the user terminal, wherein
the analysis unit
receives, when the analysis for the teeth of the user is requested, a first image obtained by photographing the teeth of the user at a first speed from the tooth photographing device to perform a first analysis, and
receives, when a doubtful area suspected of a dental disease is present in a specific tooth among the teeth of the user as a result of the first analysis, a second image obtained by photographing the doubtful area at a second speed slower than the first speed from the tooth photographing device to perform a second analysis.

2. The tooth analysis server of claim 1, further comprising:
a photographing assistant unit, wherein,
when the second image is photographed using the tooth photographing device, the photographing assistant unit provides the user terminal with an assistant photographing area for assisting the photographing such that the analysis unit analyzes the doubtful area being photographed using the tooth photographing device.

3. The tooth analysis server of claim 1, further comprising:
an advertisement transmitting unit, wherein
the advertisement transmitting unit transmits an advertisement to the user terminal during analyzing a state of the teeth of the user by the analysis unit.

4. The tooth analysis server of claim 1, further comprising:
a hospital recommendation unit, wherein,
when the analysis unit analyzes that a specific tooth has a dental disease as a result of the second analysis, the result providing unit provides an expected treatment amount for treating the dental disease to the user terminal, and
the hospital recommendation unit provides hospital information for treating the disease within the expected treatment amount to the user terminal.

5. A tooth analysis terminal comprising:
a terminal communication unit for communicating with at least one of a tooth photographing device for photographing teeth, and a tooth analysis server for analyzing a state of the teeth of the user based on an artificial intelligence learning result; and
a display unit, when an analysis of teeth of a user is requested from the user, for outputting an analysis result obtained by analyzing an image of the teeth of the user photographed through the tooth photographing device, by the artificial intelligence learning result, wherein
the display unit
outputs a first analysis result obtained by analyzing a first image of the user's teeth photographed at a first speed is analyzed by the artificial intelligence learning result, and
further outputs a first analysis result obtained by analyzing a second image, when a doubtful area suspected of a dental disease is present in a specific tooth among the teeth of the user as a result of the first analysis, in which the doubtful area is photographed at a second speed slower than the first speed, by the artificial intelligence learning result.

6. A dental analysis program stored in a medium to execute:
receiving a request for analysis of teeth of a user from the user;
receiving a first image of the user's teeth photographed at a first speed;
providing the user with a first analysis result in which the first image is analyzed by an artificial intelligence learning result;
receiving a second image, when a doubtful area suspected of a dental disease is present in a specific tooth among the user's teeth as a result of the first analysis, in which the doubtful area is photographed at a second speed slower than the first speed; and
providing the user with a second analysis result in which the second image is analyzed by the artificial intelligence learning result.
